# EUROPÄISCHE PATENTSCHRIFT

(11) **EP 2 811 911 B1**
(45) Veröffentlichungstag und Bekanntmachung des Hinweises auf die Patenterteilung: **31.10.2018**
(21) Anmeldenummer: 13703722.2
(22) Anmeldetag: 05.02.2013
(51) Int. Cl.: A61B 17/80, A61B 17/86, A61B 17/88, A61B 17/17, A61B 17/00, A61B 90/00

(54) **BEFESTIGUNGSVORRICHTUNG FÜR CHIRURGISCHE HALTESYSTEME**
FASTENING DEVICE FOR SURGICAL HOLDING SYSTEMS
DISPOSITIF DE FIXATION POUR SYSTÈMES DE MAINTIEN CHIRURGICAUX

(30) Priorität: 06.02.2012 EP 12405017
(43) Veröffentlichungstag der Anmeldung: 17.12.2014
(60) Teilanmeldung: 18197347.0
(73) Patentinhaber: Creaholic S.A., 2503 Biel (CH)
(72) Erfinder: VAUCHER, Vincent, CH-2743 Eschert (CH); MÜLLER, Markus A., 8064 Zürich (CH)
(74) Vertreter: Frei Patent Attorneys
(86) Internationale Anmeldenummer: PCT/CH2013/000022
(87) Internationale Veröffentlichungsnummer: WO 2013/116952

(56) Entgegenhaltungen:
- EP-A2- 1 346 697
- WO-A2-2009/017656
- US-A- 5 464 407
- US-A1- 2005 277 940
- US-A1- 2006 264 936
- US-A1- 2012 022 600

## Beschreibung

Die Erfindung bezieht sich auf das Gebiet der chirurgischen Haltesysteme und insbesondere auf eine Befestigungsvorrichtung für chirurgische Haltesysteme, ein chirurgisches Halteelement und ein Werkzeug für chirurgische Haltesysteme gemäss dem Oberbegriff der entsprechenden unabhängigen Patentansprüche.

Chirurgische Haltesysteme, beispielsweise zum Fixieren von gebrochenen Knochen bestehen oft aus Platten, welche mittels Schrauben in den Knochen befestigt sind. Die Platten weisen Öffnungen auf, in welche die Schrauben unter verschiedenen Winkeln eingebracht werden. Aus der WO 2010/121388 A1 derselben Anmelderin ist ein chirurgisches Haltesystem bekannt, bei welchem ein Gelenkkopf einer Schraube mit einer Gelenkpfanne in einer Platte ein Kugelgelenk bildet. Die Orientierung der Schraube in der Gelenkpfanne ist mittels eines exzentrisch ausgebildeten Klemmringes fixierbar. Der Klemmring ist dazu in der Platte drehbar gelagert und verklemmt sich bei Verdrehung gegen den Gelenkkopf der Schraube.

Dabei tritt das Problem auf, dass beim Verklemmen, bedingt durch die Geometrie des Kugelgelenkes und des Klemmringes, sich genau zwei Kontaktpunkte zwischen einerseits dem Gelenkkopf und andererseits der Gelenkpfanne und dem Klemmring ergeben. Dies ist eine Folge der nichtidealen Form dieser Teile, bedingt durch Fertigungstoleranzen. Es ist dann zwar möglich, dass sich durch weiteres, starkes Verspannen des Klemmringes die Teile plastisch verformen und weitere Kontaktpunkte entstehen. Geschieht dies aber nicht, so definieren die zwei Kontaktpunkte eine Achse, um welche sich der Gelenkkopf bezüglich der Gelenkpfanne drehen kann, so dass die Verbindung nicht unter allen Umständen richtig arretiert ist.

### DARSTELLUNG DER ERFINDUNG

Es ist deshalb Aufgabe der Erfindung, eine Befestigungsvorrichtung für chirurgische Haltesysteme und ein chirurgisches Halteelement der eingangs genannten Art zu schaffen, welche die oben genannten Nachteile beheben.

Diese Aufgabe löst eine Befestigungsvorrichtung für chirurgische Haltesysteme mit den Merkmalen des unabhängigen Anspruchs 1. Weitere bevorzugte Ausführungsformen gehen aus den abhängigen Patentansprüchen hervor.

Eine weitere Aufgabe, jedoch nicht im Rahmen der vorliegenden Erfindung, ist, ein Werkzeug zu schaffen, mit welchem die Befestigungsvorrichtung sicher und einfach arretiert respektive gelöst werden kann, und auch die Schraube ein- respektive ausgeschraubt werden kann.

Die Befestigungsvorrichtung, vorgesehen für chirurgische Haltesysteme weist mindestens ein Halteelement und ein Befestigungselement auf, welche mechanisch miteinander verbunden werden können. Das Halteelement weist eine Gelenkpfanne auf, und das Befestigungselement einen Gelenkkopf. Die Gelenkpfanne und der Gelenkkopf korrespondieren vorzugsweise zumindest abschnittsweise in ihrer Form miteinander und bilden zusammen ein Kugelgelenk. Die Bewegung des Kugelgelenkes ist mittels eines Klemmelementes arretierbar. Das Klemmelement der Befestigungsvorrichtung weist einen Ring mit exzentrischer Form auf, welcher beim Verdrehen des Ringes den Gelenkkopf bezüglich der Gelenkpfanne verklemmt und/oder verspannt. Die Gelenkpfanne und eine Ringinnenfläche des Exzenter-Ringes definieren zusammen eine Gelenkinnenfläche einer Gelenkschale, in welcher der Gelenkkopf gelagert ist, wobei eine äussere Kugelfläche des Gelenkkopfes der Gelenkinnenfläche gegenüber liegt. Dabei ist, wenn der Exzenter-Ring den Gelenkkopf bezüglich der Gelenkpfanne verklemmt, durch die Form von mindestens einem der drei Elemente Gelenkpfanne, Gelenkkopf und Exzenter-Ring die Lage von drei Kontaktstellen des Kugelgelenkes, welche das Kugelgelenk arretieren, eindeutig definiert.

Dies steht im Gegensatz zu Verbindungssystemen, bei welchen beispielsweise eine glatte oder eine gerillte, profilierte oder in anderer Weise strukturierte Oberfläche an der Gelenkinnenfläche und/oder der Kugelfläche vorliegt. Solche Oberflächen führen zu einer Vielzahl von Kontaktstellen, deren Lage aber vom Zufall anhängt und somit nicht eindeutig definiert ist. Bei der hier vorgestellten Befestigungsvorrichtung sind es hingegen mindestens drei Kontaktstellen, was nicht ausschliesst, dass unter bestimmten geometrischen Verhältnissen noch eine vierte eindeutig definierte Kontaktstelle vorliegt, ohne dass eine Verformung an diesen Teilen stattgefunden hat. Ferner ist auch nicht ausgeschlossen, dass bei zunehmender Verklemmung nicht weitere Kontaktstellen entstehen, deren Lage jedoch nicht definiert ist.

Dadurch ist es möglich, die Schrauben in einem relativ frei wählbaren Winkel in den Knochen zu schrauben, und unabhängig von der genauen Position der Schrauben diese mit der Platte, dank der Dreipunktverbindung zwischen Gelenkpfanne (mit Klemmelement) und Gelenkkopf winkelstabil zu verriegeln.

Der Exzenterring ist - egal ob er am Halteelement oder am Befestigungselement drehbar angeordnet ist - in der Regel einerseits selber, bezüglich des Mittelpunktes des Kugelgelenkes, exzentrisch gelagert, also um eine Achse drehbar, welche nicht durch diesen Mittelpunkt führt. Andererseits sind eine im wesentlichen einer zylindrischen Form folgende Innenfläche des Ringes, welche mindestens abschnittsweise oder punktuell mit dem Befestigungselement in Berührung ist, und eine im wesentlichen einer zylindrischen Form folgende Aussenfläche des Ringes, welche mindestens abschnittsweise oder punktuell mit dem Halteelement in Berührung ist, nicht konzentrisch zueinander.

Gemäss einer Variante der Befestigungsvorrichtung, vorgesehen für chirurgische Haltesysteme, weist sie mindestens ein Halteelement und ein Befestigungselement auf, welche mechanisch miteinander verbunden werden können. Das Halteelement weist eine Gelenkpfanne auf, und das Befestigungselement einen Gelenkkopf Die Gelenkpfanne und der Gelenkkopf korrespondieren vorzugsweise zumindest abschnittsweise in ihrer Form miteinander und bilden zusammen ein Kugelgelenk. Die Bewegung des Kugelgelenkes ist mittels eines Klemmelementes arretierbar. Das Klemmelement der Befestigungsvorrichtung weist einen Ring mit exzentrischer Form auf, welcher beim Verdrehen des Ringes den Gelenkkopf bezüglich der Gelenkpfanne verklemmt und/oder verspannt. Die Gelenkpfanne und eine Ringinnenfläche des Exzenter-Ringes definieren zusammen eine Gelenkinnenfläche einer Gelenkschale, in welcher der Gelenkkopf gelagert ist, wobei eine äussere Kugelfläche des Gelenkkopfes der Gelenkinnenfläche gegenüber liegt. Dabei
▪ weist entweder die Gelenkinnenfläche genau zwei oder genau drei nach innen vorstehende Kontaktbereiche auf,
▪ oder weist die Gelenkaussenfläche genau zwei oder genau drei nach aussen vorstehende Kontaktbereiche auf,
wobei, wenn der Exzenter-Ring den Gelenkkopf bezüglich der Gelenkpfanne verklemmt, aber keine Verformung an diesen Teilen stattgefunden hat, genau drei Kontaktstellen oder auch genau vier Kontaktstellen zwischen der Gelenkinnenfläche und der Gelenkaussenfläche vorliegen.

Zur Terminologie sei vermerkt dass in der Regel der Begriff "Kontakibereich" (meist als vorstehender Kontaktbereich) für eine durch die Form eines Elementes - also Gelenkpfanne, Gelenkkopf oder Klemmelement - vorgegebene und somit auch am Element selber erkennbare Ausgestaltung des Elements steht. Der Begriff "Kontaktstelle" hingegen bezeichnet Punkte oder Bereiche, in welchen beim Verklemmen die Elemente aufeinander aufliegen und die grössten Kräfte übertragen werden respektive auftreten. Wo genau die Kontaktstellen auftreten ist abhängig von der gegenseitigen Anordnung der Elemente und von der Lage ihrer Kontaktbereiche. Einige der Kontaktbereiche werden zu Kontaktstellen. Umgekehrt ergeben sich einige Kontaktstellen bei Kontaktbereichen, andere Kontaktstellen ergeben sich typischerweise dadurch, indem ein oder insbesondere zwei Kontaktbereiche von einer gegenüberliegenden Seite des Gelenkes her auf die Elemente drücken.

In einer Ausführungsform ist der Exzenter-Ring am Halteelement drehbar angeordnet und wird bei Verdrehung des Exzenter-Ringes bezüglich des Halteelementes der Exzenter-Ring selber oder ein vom Exzenter-Ring bewegtes Zwischenelement gegen die Gelenkaussenfläche gedrückt, respektive gegen den Gelenkkopf

Eine äussere Fläche des Ringes, entlang welcher der Ring im Halteelement drehbar ist, weist eine rotationssymmetrische Form auf. Die Ringinnenfläche bildet eine Kontaktfläche mit dem Gelenkkopf und kann mindestens annähernd einen Abschnitt einer Kugelfläche bilden. Beim Verdrehen des Klemmelementes drückt die Kontaktfläche gegen die kugelabschnittsförmige Aussenfläche des Gelenkkopfes.

Somit liegen im Bereich der Ringinnenfläche und der Gelenkpfanne bezüglich des Gelenkkopfes zwei oder drei gegenüber den übrigen Punkten dieses Bereiches nach innen vorstehende Kontaktbereiche vor, und/oder es liegen an der Kugelfläche zwei oder drei gegenüber den übrigen Punkten der Kugelfläche nach aussen vorstehende Kontaktbereiche vor.

In einer Ausführungsform ist der Exzenter-Ring am Befestigungselement drehbar angeordnet und wird bei Verdrehung des Exzenter-Ringes bezüglich des Befestigungselementes der Exzenter-Ring selber oder ein vom Exzenter-Ring bewegtes Zwischenelement gegen die Gelenkinnenfläche gedrückt, respektive gegen die Gelenkpfanne

Gemäss verschiedenen Ausführungsformen entstehen beispielsweise zwei der definierten Kontaktstellen an zwei entsprechenden Kontaktbereichen durch Verformen des Exzenter-Ringes, insbesondere an zwei Seiten oder Enden (entlang des Umfangs betrachtet) einer entlang des Umfanges des Exzenter-Ringes verlaufenden biegeschwachen Stelle. Die andernorts zur vorliegenden Erfindung beschriebenen Ausführungsformen mit vorstehenden Kontaktbereichen am Exzenter-Ring lassen sich grundsätzlich auch mit dieser Variante mit Verformung des Exzenter-Ringes realisieren.

Gemäss verschiedenen Ausführungsformen weist die Gelenkinnenfläche genau zwei oder genau drei nach innen vorstehende Kontaktbereiche auf und die Kugelfläche keine nach aussen vorstehende Kontaktbereiche. Diese Kontaktbereiche der Gelenkinnenfläche können am Ring und/oder an der Gelenkpfanne ausgebildet sein, wobei die Gesamtzahl der vorstehenden Kontaktbereiche kleiner oder gleich drei oder vier oder fünf sein soll.

Allgemein ist vorteilhaft, wenn die definierten Kontaktstellen, die gemäss den unterschiedlichen Ausführungsformen gebildet werden können, in einer Projektion in eine Ebene, in welcher sich das Halteelement erstreckt, betrachtet, zumindest annähernd gleichmässig an den Gelenkflächen des Kugelgelenkes verteilt sind.

Es sind im Allgemeinen beispielsweise die folgenden Kombinationen von Kontaktbereichen und sich daraus ergebenden Kontaktstellen möglich:
1. **drei** vorstehende Kontaktbereiche am Ring, **kein** vorstehender Kontaktbereich an der Gelenkpfanne. Beim Verklemmen wird, abhängig von toleranzbedingten Ungenauigkeiten, eine Kontaktstelle der Gelenkpfanne die Klemmkraft, welche durch die drei vorstehenden Kontaktbereiche am Ring in den Gelenkkopf eingeleitet wird, aufnehmen. Zwischen dieser Kontaktstelle und den zwei Kontaktbereichen des Ringes, welche dieser Kontaktstelle gegenüber liegen, stellt sich dann die Situation ein, wie sie sich auch in der folgenden Kombination ergibt, d.h. es liegen im Ergebnis, beim Verklemmen, zwei Kontaktstellen am Ring und eine Kontaktstelle an der Gelenkpfanne vor.
2. **zwei** vorstehende Kontaktbereiche am Ring, **kein** vorstehender Kontaktbereich an der Gelenkpfanne. Die beiden Kontaktbereiche am Ring drücken beim Verklemmen den Gelenkkopf gegen eine Kontaktstelle, welche auf der Winkelhalbierenden der Verbindungslinien der beiden Kontaktbereiche mit der Mitte des Gelenkkopfes, aber den beiden Kontaktbereichen gegenüber liegt. Der Ort dieser Kontaktstelle ist also, wenn der Ring in Klemmposition ist, durch die Lage der beiden Kontaktbereiche definiert und bildet mit den beiden Kontaktbereichen die Dreipunktverbindung zwischen Gelenkpfanne/Ring und Gelenkkopf. Im Ergebnis liegen also zwei Kontaktstellen am Ring und eine Kontaktstelle an der Gelenkpfanne vor.
3. **zwei** vorstehende Kontaktbereiche am Ring, ein vorstehender Kontaktbereich an der Gelenkpfanne. Der letztere ist am Ort anzuordnen, wo die oben beschriebene Kontaktstelle liegt. Im Ergebnis liegen also auch hier zwei Kontaktstellen am Ring und eine Kontaktstelle an der Gelenkpfanne vor.
4. **ein** vorstehender Kontaktbereich am Ring, **zwei** vorstehende Kontaktbereiche an der Gelenkpfanne. Analog zur **dritten** Kombination ist der eine Kontaktbereich am Ring dort anzuordnen, wo die Winkelhalbierende der Verbindungslinien der beiden Kontaktbereiche der Gelenkpfanne mit der Mitte des Gelenkkopfes, an der gegenüberliegende Seite die Ringinnenfläche durchstösst. Im Ergebnis liegen also zwei Kontaktstellen an der Gelenkpfanne und eine Kontaktstelle am Ring vor.
5. **kein** vorstehender Kontaktbereich am Ring, **zwei** vorstehende Kontaktbereiche an der Gelenkpfanne. Analog zur **zweiten** Kombination liegt der eine Kontaktbereich am Ring dort, wo die Winkelhalbierende der Verbindungslinien der beiden Kontaktbereiche der Gelenkpfanne mit der Mitte des Gelenkkopfes, an der gegenüberliegende Seite, die Ringinnenfläche durchstösst. Im Ergebnis liegen also auch hier zwei Kontaktstellen an der Gelenkpfanne und eine Kontaktstelle am Ring vor.
6. **kein** vorstehender Kontaktbereich am Ring, **drei** vorstehende Kontaktbereiche an der Gelenkpfanne. Analog zur **ersten** Kombination ergibt sich durch Zufall, zu welchem Paar der drei Kontaktbereiche der Gelenkpfanne zuerst eine Kontaktstelle an der Ringinnenfläche auftritt. Mit diesem Paar ergibt sich dann die Situation gemäss der vorangehenden Kombination, also zwei Kontaktstellen an der Gelenkpfanne und eine Kontaktstelle am Ring.

Bei allen Kombinationen besteht jeweils eine weitere Variante darin, dass die drei Kontaktbereiche oder Kontaktstellen, welche die Klemmkraft aufnehmen, nicht auf einer Ebene durch den Mittelpunkt der Kugelfläche liegen. In diesem Fall liegt der Ort der Kontaktstelle, der durch zwei gegenüberliegende vorstehende Kontaktbereiche definiert ist, jeweils in einer Ebene, welche durch die jeweilige Winkelhalbierende führt und normal zur Verbindungslinie der beiden vorstehenden Kontaktbereiche verläut. Es kann geschehen, dass der Gelenkkopf an einem oder mehreren dieser Kontaktbereiche oder Kontaktstellen abrutscht, bis sich eine vierte Kontaktstelle ergibt. Auch kann sich ergeben, dass bei zunehmender Verklemmung der Elemente und bei Deformation mindestens eines der Elemente eine vierte Kontaktstelle ergibt. Mit einer vierten Kontaktstelle kann die Verteilung der Kontaktstellen derart sein, dass
▪ zwei Kontaktstellen am Ring und zwei Kontaktstellen an der Gelenkpfanne vorliegen; oder
▪ eine Kontaktstelle am Ring und drei Kontaktstellen an der Gelenkpfanne vorliegen.

Die Kontaktbereiche am Ring können so angeordnet sein, dass Verbindungslinien der beiden Kontaktbereiche zur Mittelachse des Exzenter-Ringes
▪ wenn eine Kontaktstelle am Ring vorliegt, einen Winkel von weniger als 180° bilden; und
▪ wenn zwei Kontaktstellen am Ring vorliegen, einen Winkel von weniger als 170° und insbesondere weniger als 100° bilden.

Die Kontaktbereiche an der Gelenkpfanne können im wesentlichen gleichmässig am Umfang einer Durchführung für das Befestigungselement angeordnet sein, wobei die Durchführung durch das Halteelement und die Gelenkpfanne hindurch führt.

In einer Ausführungsform ist der Exzenter-Ring am Befestigungselement drehbar angeordnet und weist der Gelenkkopf ein Zwischenelement auf, welches durch Verdrehen des Exzenter-Ringes gegen die Gelenkpfanne des Halteelementes drückbar ist und dadurch eine erste Kontaktstelle bildet und das Kugelgelenk arretiert. Dabei weist der Gelenkkopf mindestens zwei Segmente auf, welche dem Zwischenelement bezüglich des Gelenkkopfes gegenüberliegend angeordnet sind, wobei jedes der mindestens zwei Segmente einen Kontaktbereich und damit eine weitere Kontaktstelle des Kugelgelenkes definiert respektive bildet. Der Ring berührt also das Halteelement nicht, sondern drückt nur über das Zwischenelement gegen das Halteelement. Es sind somit wiederum drei (oder beispielsweise vier) Kontaktstellen eindeutig definiert.

Umgekehrt ist, gemäss einer analogen Ausführungsform, der Exzenter-Ring am Halteelement drehbar angeordnet und weist das Halteelement ein Zwischenelement auf, welches durch Verdrehen des Exzenter-Ringes gegen die Kugelfläche des Befestigungselementes drückbar ist und dadurch eine erste Kontaktstelle bildet und das Kugelgelenk arretiert. Dabei weist die Gelenkpfanne mindestens zwei vorstehende Kontaktbereiche auf, welche dem Zwischenelement bezüglich des Zentrums der Gelenkpfanne gegenüberliegend angeordnet sind, wobei jedes der mindestens zwei vorstehenden Kontaktbereiche eine weitere Kontaktstelle des Kugelgelenkes definiert respektive bildet. Der Ring berührt also das Befestigungselement nicht, sondern drückt nur über das Zwischenelement gegen das Befestigungselement.

In einer Ausführungsform ist der Exzenter-Ring am Befestigungselement drehbar angeordnet und ist ein Abschnitt des Exzenter-Ringes durch Verdrehen des Exzenter-Ringes gegen die Gelenkpfanne des Halteelementes drückbar und bildet dadurch eine erste Kontaktstelle und arretiert das Kugelgelenk. Dabei weist der Gelenkkopf mindestens zwei Segmente auf, welche der ersten Kontaktstelle bezüglich des Gelenkkopfes gegenüberliegend angeordnet sind, wobei jedes der mindestens zwei Segmente einen Kontaktbereich und damit eine weitere Kontaktstelle des Kugelgelenkes definiert respektive bildet. Dadurch sind wiederum drei (oder beispielsweise vier) Kontaktstellen eindeutig definiert.

Das Kugelgelenk kann dadurch realisiert sein, dass sowohl der Gelenkkopf als auch die Gelenkpfanne - mit Ausnahme der Kontaktbereiche - zueinander passende sphärische Flächen aufweisen. In einer weiteren Ausführungsform der Erfindung ist die Gelenkpfanne nur in einem Teilbereich sphärisch, und in einem übrigen Bereich sich gegen das Klemmelement hin öffnend, beispielsweise konisch, ausgebildet. Der Teilbereich liegt dabei an der Seite des Haltelementes, welche dem Klemmelement gegenüber liegt, der übrige Bereich liegt zwischen dem Teilbereich und dem Klemmelement. In einer dritten Ausführungsform ist die Gelenkpfanne vollständig sich gegen das Klemmelement hin öffnend, vorzugsweise konisch, ausgebildet. Auch in der zweiten und der dritten Ausführungsform der Erfindung wird aufgrund der sphärischen Form des Gelenkkopfes mit der Gelenkpfanne eine Kugelgelenkverbindung gebildet und können die vorstehenden Kontaktbereiche entsprechend angeordnet sein.

Gemäss einer weiteren Ausführungsform ist die Gelenkpfanne nur durch drei Kontaktbereiche gebildet, und kommen keine anderen Punkte der Gelenkpfanne je in Berührung mit dem Gelenkkopf.

In einer nicht erfindungsgemäßen Ausführungsform ist das Befestigungselement im wesentlichen rotationssymmetrisch bezüglich einer Längsachse des Befestigungselementes gestaltet, wobei eine Ebene, welche durch die drei definierten Kontaktstellen führt, diese Längsachse nicht enthält. Wäre nämlich die Längsachse in dieser Ebene, welche normal oder annähernd normal zur Ebene des Halteelementes verläuft, so wäre der Abstand zwischen einzelnen der Kontaktstellen durch die Dicke des Halteelements begrenzt. Durch die Nähe der Kontaktstellen wiederum wäre das Haltemoment relativ klein. Um ein möglichst hohes Haltemoment bei Arretierung des Kugelgelenkes zu erzielen, kann die erwähnte Ebene der Kontaktstellen möglichst parallel zur Ebene des Halteelementes sein. Dies ist, falls der Ring am Halteelement angebracht ist, im Wesentlichen auch die Ebene des Ringes oder Ring-Ebene (genauer gesagt: einer Ebene, welche normal zur Rotationsachse des Exzenter-Ringes im Halteelement liegt).

In einer nicht erfindungsgemäßen Ausführungsform erstreckt sich das Halteelement in einer Ebene, und weist eine Ebene, welche durch die drei definierten Kontaktstellen führt, einen Winkel von maximal **45** Grad zur Ebene des Halteelementes oder zur Ring-Ebene des Exzenter-Ringes.

In einer Ausführungsform beträgt der kleinste Abstand zwischen den drei definierten Kontaktstellen mindestens das **0.8**-fache des Durchmessers des Gelenkkopfes.

In einer Ausführungsform weist das Halteelement oder das Befestigungselement (je nachdem, an welchem der Exzenter-Ring angeordnet ist) ein Einrastelement zur einrastenden Festhalten des Exzenter-Ringes bezüglich Verdrehung gegenüber dem Halteelement respektive Befestigungselement auf, Damit kann der Exzenter-Ring in einer "offenen" Stellung geliefert und gehalten werden, und muss beim Einsetzen des Befestigungselementes nicht zuerst in diese Stellung gebracht werden. Das Einrastelement kann eine Ausformung an einem der beteiligten Elemente sein, oder ein separates Teil sein. Beim Lösen des Ringes aus der eingerasteten Lage gibt beispielsweise der Ring elastisch nach.

Die Aussenseite des Klemmelementes kann einen Absatz und/oder einen Konus aufweisen der mit der Form einer Ringnut im Halteelement korrespondiert. Das Klemmelement kann eine oder mehrere Führungen aufweisen, die für den Eingriff des Werkzeuges geeignet sind, um eine Verklemmungsbewegung vorzunehmen. Beispielsweise sind dies eine oder zwei oder mehr hinterschnittige Öffnungen zum Einführen eines Werkzeuges zum Verdrehen des Exzenter-Ringes.

An dem chirurgischen Halteelement sind eine oder mehrere Gelenkpfannen mit korrespondierenden Ringnuten ausgebildet.

Grundsätzlich kann die beschriebene Befestigungsvorrichtung auch in anderen Anwendungen eingesetzt werden, beispielsweise im Maschinenbau, für Halterungen und Stative und dergleichen. Beispielsweise kann sie im Holzbau oder allgemein bei der Verbindung von miteinander zu verklebenden Teilen verwendet werden, indem zwei Teile nach dem Anbringen des eines Klebstoffes mittels der Befestigungsvorrichtung miteinander verschraubt und mit einer Vorspannung gegeneinander gezogen werden. Wenn die Verklebung ausgehärtet und stabil ist, können die Elemente der Befestigungsvorrichtung entfernt werden.

Ein **Werkzeug,** das nicht Teil der vorliegenden Erfindung ist, beispielsweise zum Fixieren, Arretieren und Lösen einer Befestigungsvorrichtung wie oben beschrieben, weist auf
▪ einen inneren Schaft mit einer inneren Werkzeugspitze,
▪ einen äusseren Schaft mit einer äusseren Werkzeugspitze,
wobei der innere Schaft innerhalb des äusseren Schaftes angeordnet ist und bezüglich des äusseren Schaftes entlang einer Längsachse der beiden Schäfte verschiebbar ist.

In einer Ausführungsform ist das Werkzeug werkzeugfrei in mindestens zwei Einheiten zerlegbar, indem mindestens der innere Schaft und damit verbundene weitere Teile als erste Einheit vom äusseren Schaft und damit verbundenen weiteren Teilen als zweite Einheit werkzeugfrei trennbar und wieder verbindbar ist.

In einer Ausführungsform ist das Werkzeug zerlegbar, indem die beiden Einheiten voneinander trennbar sind, indem zunächst die beiden Einheiten durch Verschiebung entlang einer Längsachse des Werkzeuges in eine bestimmte Position zu bringen sind, und dann die beiden Einheiten nach Drehen um die Längsachse voneinander abziehbar sind.

In einer Ausführungsform des Werkzeugs ist eine der Werkzeugspitzen dazu ausgebildet, sich mit einem damit zu drehenden Element zu verhaken, wobei der der Werkzeugspitze entsprechende Schaft biegeelastisch und drehsteif ausgebildet ist. Wenn die Werkzeugspitze mit dem zu drehenden Element verhakt ist, kann das Werkzeug beim Anbringen eines Drehmomentes nicht abrutschen. Dies ist bei chirurgischen Arbeiten wichtig und vorteilhaft. Nachteilig kann dabei aber sein, dass ein Kippen der Längsachse des Werkzeuges ein Moment auf das zu drehende Element ausüben kann. Aus diesem Grunde ist das biegeelastische Abschnitt des Schaftes vorgesehen.

In einer Ausführungsform des Werkzeugs ist es die äussere Werkzeugspitze, die dazu ausgebildet ist, sich mit einem damit zu drehenden Element zu verhaken, und somit ist es der äussere Schaft, welcher in einem elastischen Bereich biegeelastisch und drehsteif ausgebildet ist. Damit der äussere Schaft sich biegen kann, sollte im selben Bereich entlang der Länge des äusseren Schaftes entweder der innere Schaft selber auch biegeelastisch sein, oder Raum für die Biegung des äusseren Schaftes lassen. Letzteres kann beispielsweise geschehen, indem der innere Schaft so weit nach innen gezogen wird, dass dieser Bereich ganz frei ist, oder indem der innere Schaft, wenn der äussere Schaft in Arbeitsposition ist, an der Stelle dieses elastischen Bereiches eine Verjüngung aufweist.

In einer Ausführungsform des Werkzeugs weist es einen asymmetrisch wirkenden Drehmomentbegrenzer auf, insbesondere für den äusseren Schaft.

Im folgenden wird der Erfindungsgegenstand anhand von bevorzugten Ausführungsbeispielen, welche in den beiliegenden Zeichnungen dargestellt sind, näher erläutert. Es zeigen jeweils schematisch:
Figuren 1 bis 4 eine Befestigungsvorrichtung und deren Einzelteile, mit Kontaktbereichen am Ring;
Figur 5 eine alternative Ausführungsform eines Klemmringes;
Figuren 6 bis 11 eine Befestigungsvorrichtung und deren Einzelteile in einer Ausführungsform mit Kontaktbereichen am Halteelement;
Figuren 12 bis 15 eine Ausführungsform mit Kontaktbereichen am Gelenkkopf;
Figuren 16 bis 19 eine andere Ausführungsform mit Kontaktbereichen am Gelenkkopf;
Figuren 20 bis 24 eine nicht erfindungsgemäße Befestigungsvorrichtung mit einem Schraubring;
Figuren 25 bis 27 nicht erfindungsgemäße Bohrbuchsen zur Verwendung in der Befestigungsvorrichtung;
Figur 28 ein nicht erfindungsgemäßes Werkzeug zum Drehen des Rings wie auch des Befestigungselementes, mit zurückgezogenem Ringwerkzeug;
Figur 29 das Werkzeug mit vorgeschobenem Ringwerkzeug;
Figur 30 das Werkzeug, zum Reinigen zerlegt;
Figur 31 eine Explosionszeichnung des Werkzeugs; und
Figur 32 bis 34 ein Funktionsprinzip eines nicht erfindungsgemäßen Drehmomentbegrenzers.

Grundsätzlich sind in den Figuren gleiche oder gleich wirkende Teile mit gleichen Bezugszeichen versehen.

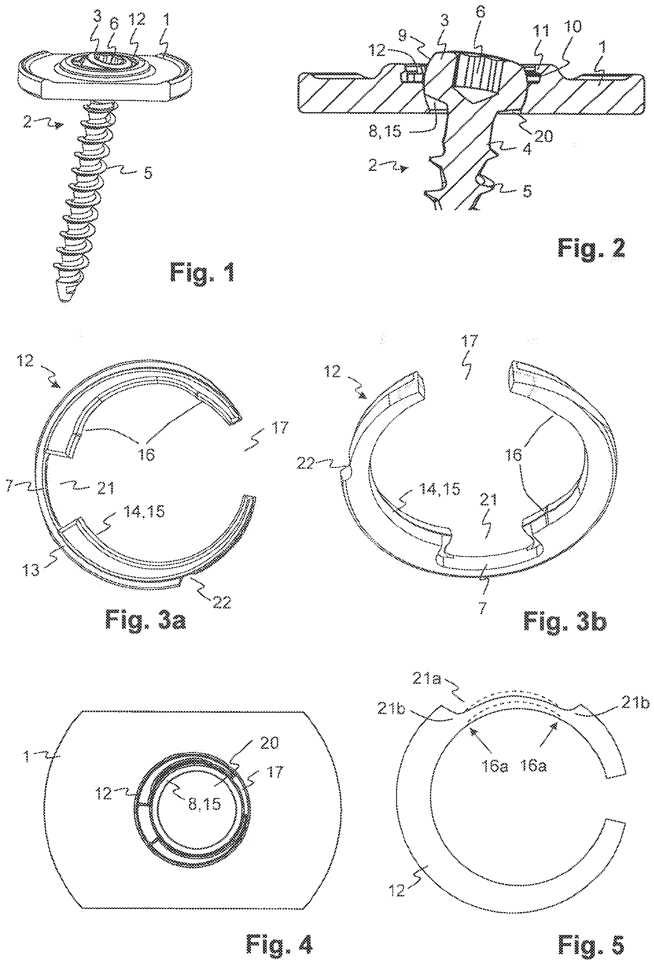

**Figur 1** zeigt eine Befestigungsvorrichtung mit einem Halteelement 1, einem Befestigungselement 2 sowie einem Klemmelement 12. **Figur 2** zeigt die verschiedenen Elemente in einer Schnittzeichnung. Das Befestigungselement 2 mit einem Gelenkkopf 3 ist in einem Halteelement 1, das eine Aufnahme für das Befestigungselement 2 mit einer Gelenkpfanne 8 aufweist, eingelegt. Das Befestigungselement 2 weist eine Aufnahme 6 für ein Werkzeug sowie einen Schaft 4 auf. Der Schaft 4 führt durch eine Durchführung 20 des Halteelementes 1, Er ist beispielsweise mit einem Gewinde 5 versehen und kann in ein zu fixierendes oder stabilisierendes Substrat, beispielsweise in einen Knochen, eingeschraubt werden. Durch das Kugelgelenk aus Gelenkpfanne 8 und Gelenkkopf 3 ist das Befestigungselement 2 im Halteelement 1 drehbar beweglich. Das Halteelement 1 ist aber nicht translatorisch entlang des Befestigungselementes 2 bewegbar. Das Kugelgelenk ist durch ein Klemmelement oder einen Ring 12 feststellbar oder fixierbar. Der Ring weist eine Öffnung 17 auf, sowie gegenüberliegend eine biegeschwache Stelle 7, die für ein vereinfachtes Montieren des Ringes 12 in eine umlaufende Ringnut 10 des Halteelementes 1 vorgesehen sind.

**Figur 3** zeigt den Ring 12 alleine in zwei verschiedenen Ansichten **3a** und **3b,** und **Figur 4** den Ring 12 eingesetzt in das Halteelement 1. Mittels der Öffnung 17 am Klemmelement respektive Ring 12 wird ein Aufweiten und ein Zusammendrücken des Ringes 12 ermöglicht. Somit kann das Klemmelement 12 zusammengedrückt und in die Ringnut 10 eingelegt werden. Durch einen Absatz 13 am Ring und eine passende Schulter 11 an der Ringnut 10 wird das Klemmelement 12 gegen Herausspringen gesichert. Anstelle des Absatzes oder zusätzlich können Ring 12 und Ringnut 10 auch zueinander korrespondierend konisch ausgebildet sein. An der Ringinnenfläche 14 liegen Kontaktbereiche 16 vor, welche gegenüber den anderen Bereichen der Ringinnenfläche 14, die Teil einer Kugelinnenfläche 15 zur Lagerung des Gelenkkopfes 3 sind, nach innen vorstehen.

Die grundsätzliche Funktionsweise der Verriegelung ist in der eingangs erwähnten WO 2010/121388 A1 im Detail beschrieben. Beispielsweise ist die Ringnut 10 gegenüber dem Zentrum des Gelenkkopfes 3 und gegebenenfalls auch einer Längsachse des Befestigungselementes 2 exzentrisch angeordnet. Durch Verdrehen des Klemmelementes 12 verschiebt sich das Zentrum einer Ringinnenfläche 14 des Klemmelementes 12 und arretiert somit den Gelenkkopf 3 gegenüber der Gelenkpfanne 8 respektive löst ihn.

Für alle Ausführungsformen gilt: Die Kombination von Gelenkpfanne 8 und Ringinnenfläche 14 des Klemmringes 12 bildet eine Gelenkschale mit einer inneren Fläche oder Gelenkinnenfläche, die eine Kugelinnenfläche 15 sein kann, in welcher der Gelenkkopf 3 gelagert ist. Indem die Ringinnenfläche 14 und/oder die Gelenkpfanne 8 vorstehende Kontaktbereiche 16 aufweisen, liegen klar definierte Bereiche vor, an denen beim Verklemmen des Ringes 12 eine Klemmkraft auf den Gelenkkopf 3 ausgeübt wird. Diese Bereiche definieren mindestens drei Punkte, an welchen der Gelenkkopf 3 gehalten ist und somit eindeutig seine Orientierung definiert ist.

Für die Ausführungsform der Figuren 1-4 gilt, dass die vorstehenden Kontaktbereiche 16 beim Verklemmen vor den anderen Bereichen der Ringinnenfläche 14 als erste in Berührung mit der Kugelfläche 9 des Gelenkkopfes 3 kommen und eine Kraft auf den Gelenkkopf 3 ausüben. Dadurch wird der Gelenkkopf 3 gegen einen gegenüberliegenden Punkt der Gelenkinnenfläche respektive Kugelinnenfläche 15 an der Gelenkpfanne 8 gedrückt, welcher einen dritten Punkt als weitere Kontaktstelle zur Fixierung des Gelenkkopfes 3 bildet. Falls der Gelenkkopf 3 an diesem Punkt abrutscht (wobei er beispielsweise um eine Verbindungslinie der beiden Kontaktbereiche 16 dreht, kann er gegen einen vierten Punkt der Kugelinnenfläche 15 stossen, der die Bewegung des Gelenkkopfes 3 endgültig begrenzt. Mit dieser Fixierung über drei oder allenfalls vier Punkte (ausgeformte Kontaktbereiche und daraus resultierende Kontaktstellen) ist die Orientierung des Gelenkkopfes 3 und damit des Befestigungselementes 2 sicher definiert und wird durch die Klemmkraft an den Kontaktbereichen respektive Kontaktstellen fixiert.

**Figur 5** zeigt einen Ring 12 in einer anderen Ausführungsform. Dieser weist eine äussere Öffnung 21a auf, und entsprechend einen dünneren, biegeschwachen Bereich des Ringes (bezüglich Biegung innerhalb der Ebene des Ringes). Die äussere Öffnung 21a ragt also von der Aussenseite in Richtung der Mitte des Ringes in diesen hinein. Die kann eine regelmässige Gelenkinnenfläche oder Kugelinnenfläche 15 ohne vorstehende Ausformungen sein. Beim Verklemmen des Ringes 12 zwischen dem Halteelement 1 und dem Befestigungselementes 2 (in **Figur 5** nicht dargestellt) wird der Ring 12 im biegeschwachen Bereich nicht durch die Flanke der Ringnut 10 im Halteelement gestützt und verformt sich nach aussen hin (in der **Figur 5** strichliert dargestellt, der besseren Sichtbarkeit halber mit einer stark übertriebenen Verformung). In Übergangsbereichen 21b, in welchen die biegeschwache Stelle beginnt respektive aufhört, treten die grössten Druckkräfte zwischen dem Gelenkkopf 3 und der Innenfläche 14 des Ringes 12 auf. Dies entspricht zwei weiteren Kontaktbereichen 16a, welche somit den Gelenkkopf 3, zusammen mit einer sich daraus ergebenden gegenüberliegenden Kontaktstelle am Halteelement 2, an drei Punkten klemmt und hält. Die Lage der beiden Kontaktbereiche 16a wie auch der gegenüberliegenden Kontaktstelle am Halteelement 2 ist somit durch die Lage der Übergangsbereiche 21b am Ring 12 eindeutig definiert.

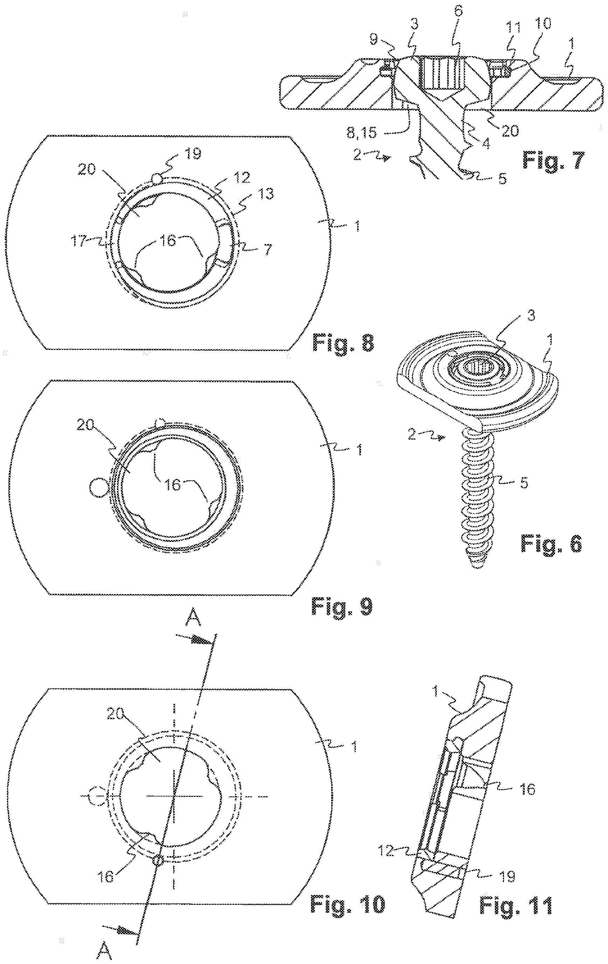

**Figur 6** zeigt eine Befestigungsvorrichtung in einer weiteren Ausführungsform, **Figur 7** in einer Schnittzeichnung. Die Elemente sind in ihrer Form und Funktion grundsätzlich gleich, mit dem Unterschied, dass am Ring 12 keine vorstehenden Kontaktbereiche 16 vorliegen, sondern am Halteelement 1 an der Gelenkpfanne 8 als Teil der Gelenkinnenfläche respektive Kugelinnenfläche 15 vorstehende Kontaktbereiche 16 vorliegen, beispielsweise im Bereich der Durchführung 20. **Figur 8** zeigt eine Aufsicht von der Seite der Gelenkpfanne 8 her auf das Halteelement 1 mit eingesetztem Ring 12, **Figur 9** dieselbe Aufsicht ohne den Ring 12. **Figur 10** zeigt eine Ansicht von unten, d.h. von der Seite der Durchführung 20 her. **Figur 11** zeigt einen Querschnitt durch ein Halteelement 1 mit eingesetztem Ring 12.
Ein Einrastelement 19 ragt in die Ringnut 10 hinein, und greift in eine korrespondierende Aussparung 22 am Absatz 13 des Ringes 12 ein (siehe **Figuren 3a** und **3b**). Das Einrastelement 19 kann wie gezeichnet aus konstruktiven Gründen durch einen eingesetzten Stift realisiert sein, kann aber gemäss anderen Ausführungsformen auch als Teil der Ringnut 10 am Halteelement 1 ausgeformt sein. Durch das Zusammenwirken von Einrastelement 19 und Aussparung am Ring kann - je nach Gestaltung der Aussparung - der Ring in einer definierten Lage einrasten und gehalten werden, in welcher das Befestigungselement 2 ohne weiteres eingesetzt werden kann. Und/oder es kann die Drehbewegung des Ringes 12 auf einen vorgegebenen Bereich begrenzt werden. Ein solches Einrastelement 19 kann natürlich auch bei der Ausführungsform der Figuren 1-4 realisiert werden.

Die Öffnung 17 und eine weitere Öffnung 21 des Ringes 12 kann hinterschnittig ausgebildet sein. Dann kann ein Werkzeug zum Verdrehen des Ringes 12 in die Öffnung 17, 21 eingeführt werden, und durch Verdrehen um die Rotationsachse des Ringes 12 mit diesem verhakt werden. Das heisst, beispielsweise ist der Bereich, durch welchen das Werkzeug in die Öffnung 17, 21 eingeführt wird, kleiner als ein weiter innen liegender Bereich der Öffnung 17, 21. Bei Zugkraft entlang der Achse des Befestigungselementes respektive des Werkzeuges rutscht das Werkzeug also nicht aus der Öffnung 17, 21. Das Werkzeug wird mittels über einen Teil der Öffnung 17, 21 vorstehende Abschnitte des Ringes zurückgehalten. Die Flanken dieser Abschnitte können schräg geformt sein, so dass beim Drehen des Werkzeuges um die Achse das Werkzeug in die Öffnung 17, 21 hinein gezogen wird. Analoges gilt für eine oder mehrere weitere Abschnitte des Ringes 12, beispielsweise für eine der Öffnung 17, 21 gegenüberliegende weitere Öffnung 21 im Bereich der biegeschwachen Stelle, wie in **Figur 3b** sichtbar. In der **Figur 3b** ist die Lage von hinterschnittigen Bereichen beidseits der Öffnung 17 durch punktierte Linien angedeutet. Eine solche hinterschnittige Öffnung 17, 21 kann bei den Ausführungsformen der Figuren 1-4 wie auch der Figuren 5-10 realisiert werden.

Für die Ausführungsform der **Figuren 6-11** gilt analog, dass die vorstehenden Kontaktbereiche 16 beim Verklemmen vor den anderen Bereichen der Gelenkpfanne 8 in Berührung mit der Kugelfläche 9 des Gelenkkopfes 3 kommen und eine Kraft auf den Gelenkkopf 3 ausüben. Dadurch wird der Gelenkkopf 3 gegen einen gegenüberliegenden Punkt der Gelenkinnenfläche oder Kugelinnenfläche 15 an der Ringinnenfläche 14 gedrückt, welcher eine weitere Kontaktstelle zur Fixierung des Gelenkkopfes 3 bildet. Mit dieser Fixierung über drei oder allenfalls vier Punkte (ausgeformte Kontaktbereiche und daraus resultierende Kontaktstellen) ist die Orientierung des Gelenkkopfes 3 und damit des Befestigungselementes 2 sicher definiert und wird durch die Klemmkraft an den Kontaktbereichen respektive Kontaktstellen fixiert.

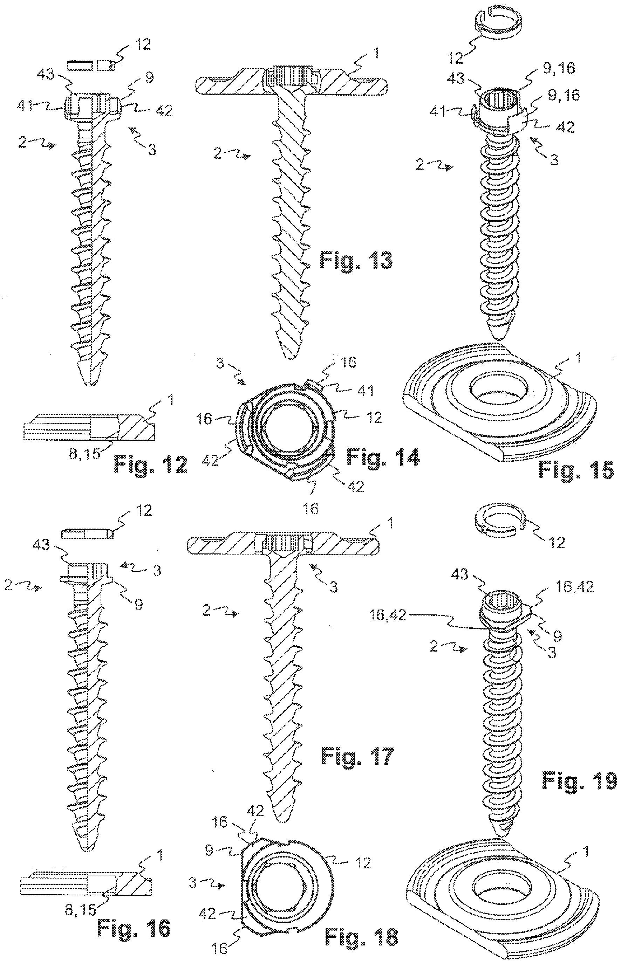

**Figuren 12** bis **15** zeigen verschiedene Ansichten und Schnitte einer Ausführungsform mit Kontaktbereichen am Gelenkkopf, wobei der Ring 12 drehbar am Gelenkkopf 3 angeordnet ist. Die Gelenkinnenfläche 15 der Gelenkpfanne 8 kann eine regelmässige Kugelinnenfläche ohne hervorstehende Ausformungen sein. Der Ring 12 ist ein Exzenterring und ist um einen Mittelteil 43 des Gelenkkopfes 3 drehbar angeordnet, um eine Achse welche exzentrisch ist, also nicht identisch zur Längsachse des Befestigungselementes 2 ist, und nicht durch den Mittelpunkt des Kugelgelenkes führt. Der Gelenkkopf 3 weist drei Segmente 42, von denen eines, im Folgenden als Zwischenelement 41 bezeichnet, so geformt sein kann, dass es bezüglich radialen Kräften elastisch nachgiebiger ist als die anderen Segmente. Die drei Segmente 42, 41 sind in etwa gleichmässig um den Umfang des Gelenkkopfes 3 verteilt angeordnet, in radialer Richtung ausserhalb des Ringes 12. An ihren Aussenseiten entspricht ihre Form der Kugelfläche 9 des Gelenkkopfes 3. Beim Verdrehen des Ringes 12 kann ein Abschnitt zunehmender Dicke des Ringes 12 zwischen den Mittelteil 43 und das Zwischenelement 41 geschoben werden, wodurch das Zwischenelement 41 in radialer Richtung nach aussen und gegen die Gelenkinnenfläche 15 des Halteelementes 1 gedrückt wird. Dadurch wird der Gelenkkopf 3 zwischen dem Zwischenelement 41 und den anderen beiden Segmenten 42 eingeklemmt und arretiert. Drei vorstehende Kontaktbereiche 16, welche die Kontaktstellen am Gelenk definieren sind also durch die Aussenseiten der Segmente 42 und des Zwischenelementes 41 definiert.

**Figuren 16** bis **19** zeigen verschiedene Ansichten und Schnitte einer anderen Ausführungsform mit Kontaktbereichen am Gelenkkopf, wobei der Ring 12 drehbar am Gelenkkopf 3 angeordnet ist. Die Gelenkinnenfläche 15 der Gelenkpfanne 8 kann eine regelmässige Kugelinnenfläche ohne hervorstehende Ausformungen sein. Der Ring 12 ist ein Exzenterring und ist um einen Mittelteil 43 des Gelenkkopfes 3 drehbar um eine Achse welche exzentrisch ist, also nicht identisch zur Längsachse des Befestigungselementes 2 ist, und nicht durch den Mittelpunkt des Kugelgelenkes führt. Der Gelenkkopf 3 weist zwei relativ kurze Segmente 42 auf. Die beiden Segmente 42 sind an zwei von drei in etwa gleichmässig um den Umfang des Gelenkkopfes 3 verteilten Stellen angeordnet, in radialer Richtung ausserhalb des Ringes 12. An ihren Aussenseiten entspricht die Form der Segmente 42 der Kugelfläche 9 des Gelenkkopfes 3. An der dritten der genannten drei Stellen kann zwischen dem Ring 12 und der Gelenkinnenfläche 15 der Gelenkpfanne 8 eine Kontaktstelle gebildet werden. Dazu kann durch Verdrehen des Ringes 12 ein Abschnitt grösserer Dicke des exzentrischen Ringes 12 gegen die Gelenkinnenfläche 15 gedrückt werden. Dadurch wird der Gelenkkopf 3 zwischen dieser Kontaktstelle und den anderen beiden Segmenten 42 eingeklemmt und arretiert. Zwei vorstehende Kontaktbereiche 16 sind also durch die Aussenseiten der beiden Segmente 42 gebildet. Sie definieren die drei Kontaktstellen am Gelenk: dies sind einerseits die beiden Kontaktbereiche 16 und andererseits die den beiden Kontaktbereichen 16 respektive Segmenten 42 gegenüberliegende Stelle am Ring 12.

Die verkürzten Segmente 42 der Ausführungsform der **Figuren 16 - 19** können auch in der Ausführungsform der **Figuren 12** - **15** verwendet werden, und umgekehrt die grösseren Segmente 42 der Ausführungsform der **Figuren 12** - **15** in der Ausführungsform der **Figuren 16** - **19****.** Bei den Ausführungsformen der **Figuren 12** - **19** kann wie bei der Ausführungsform der **Figuren 6 - 11** die Gelenkpfanne 8 vorstehende Kontaktbereiche 16 aufweisen.

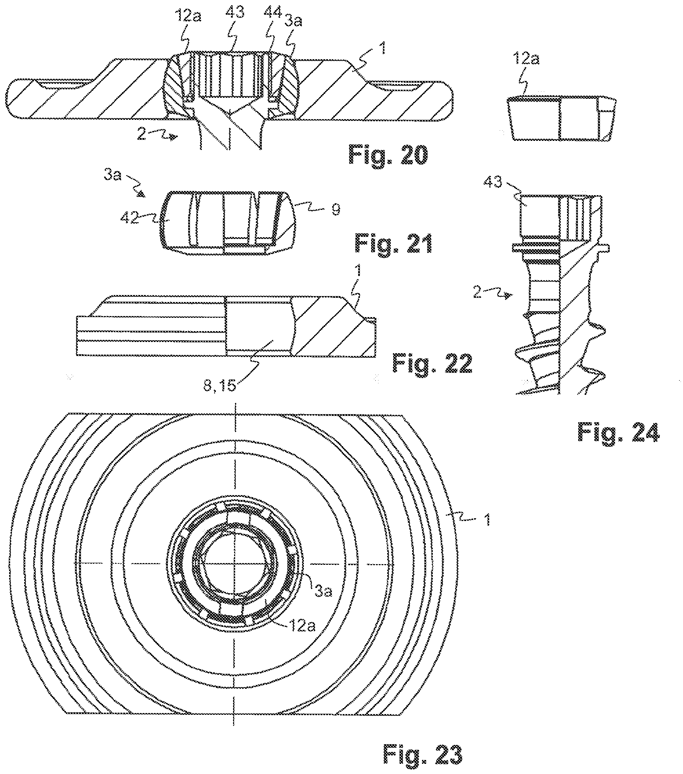

**Figuren 20** bis **24** zeigen eine Befestigungsvorrichtung, in welcher ein Schraubring 12a drehbar am Gelenkkopf 3 angeordnet ist. Der Schraubring kann um eine Achse durch den Mittelpunkt des Kugelgelenkes drehbar, also nicht exzentrisch bezüglich des Gelenkkopfes 3 angeordnet sein. Der Gelenkkopf 3 weist in Umfangsrichtung mehrere Segmente 42 auf, welche durch Schlitze voneinander getrennt sind und dadurch nach aussen gedrückt werden können. Eine Innenseite der Segmente 42 (in radialer Richtung gesehen) ist konisch geformt, und der Schraubring ist an seinem äussern Umfang als entsprechender Aussenkonus geformt. Durch Aufschrauben des Schraubringes 12a auf den Mittelteil 43 werden die Segmente 42 nach aussen und gegen die Gelenkpfanne 8 gedrückt, wodurch das Gelenk arretiert wird.

In den **Figuren 1 - 24** sind jeweils Ausschnitte eines chirurgischen Halteelementes 1 gezeigt. Ein vollständiges chirurgisches Halteelement 1 weist vorzugsweise mehrere Aufnahmen für Befestigungselemente 2 auf. Bei Anwendungen an der Wirbelsäule könnten auch Ausführungsformen mit nur einem Gelenk anwendbar sein.

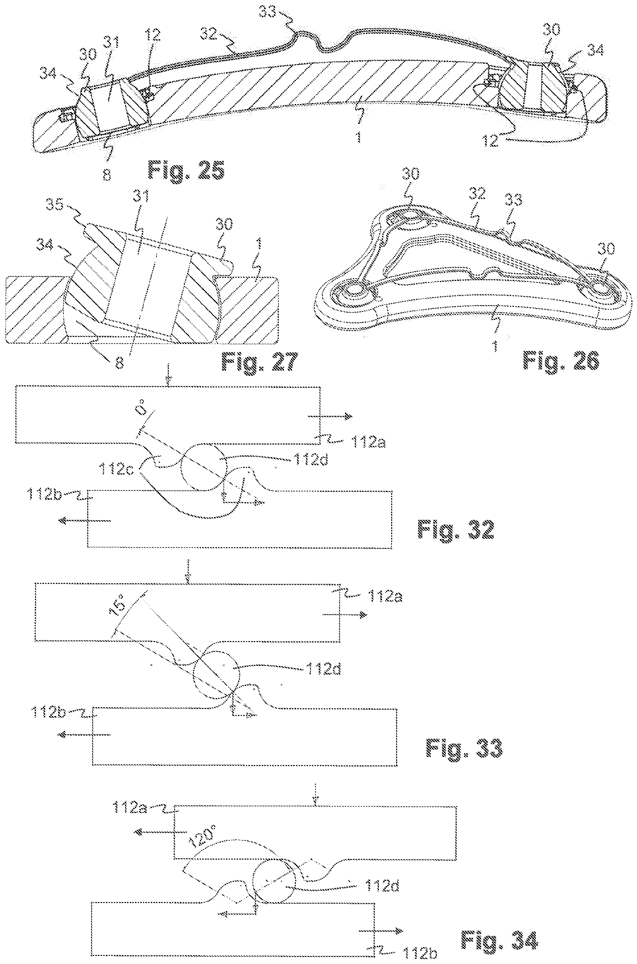

**Figuren 25** und **26** zeigen Bohrbuchsen 30 zur Verwendung in der Befestigungsvorrichtung, in einer Ansicht und einem Querschnitt. Eine Bohrbuchse 30 weist eine zylindrische Führungsöffnung 31 zur Führung eines Bohrers auf, Ein Innendurchmesser der Führungsöffnung entspricht somit einem Aussendurchmesser des vorgesehenen Bohrers. An der Aussenseite sind die Bohrbuchsen 30 gleich wie die Gelenkköpfe geformt, mit einer äusseren Kugelfläche 34, und bilden somit mit der Gelenkpfanne 8 und dem Ring 12 ein Kugelgelenk. Durch Verdrehen des Ringes 12 ist somit auch dieses Kugelgelenk arretierbar. Mehrere Bohrbuchsen 30 können mit Verbindungselementen oder Verbindungsstegen 32 miteinander verbunden sein. Diese Verbindungsstege 32 bilden eine Verliersicherung und erlauben eine einfache Handhabung eines Satzes von Bohrbuchsen 30. Die Verbindungsstege 32 können aus elastischem Material gebildet sein und können Ausgleichsbereiche 33 aufweisen, so dass die Bohrbuchsen 30 für sich alleine bewegbar sind, insbesondere zum Einstellen ihrer Orientierung in den Gelenkpfannen 8. Die Bohrbuchsen 30 sind beispielsweise aus einem im resorbierbaren, im Körper abbaubaren Material gefertigt, so dass beim Bohren entstehende Späne oder Abrieb unproblematisch sind. **Figur 27** zeigt eine Bohrbuchse 30 mit einem Anschlag 35, welcher die Verdrehung der Bohrbuchse 30 in der Gelenkpfanne 8 begrenzt. Der Anschlag 35 kann ein vorstehender Ring sein, oder mehrere ringartig angeordnete vorstehende Elemente, welch in einer Ebene normal zur Achsrichtung der Führungsöffnung angeordnet sind. Dadurch begrenzen sie eine Verdrehung der Bohrbuchse auf eine maximalen Winkelabweichung von einer Normalen zur Oberfläche des Halteelements 1.

Die Bohrbuchsen 30 werden zum Führen eines Bohrers zum Vorbohren von Löchern, typischerweise in einem Knochen, vor dem Einsetzen oder Einschrauben der Befestigungselemente oder Schrauben 2 verwendet. Dazu können die Bohrbuchsen 30 vor Verwendung in das Halteelement 1 eingesetzt werden oder bereits eingesetzt geliefert werden. Vor dem Einsatz des Halteelementes 1 können die Bohrbuchsen 30 nach Bedarf orientiert und dann mittels des Ringes 12 arretiert werden. Dann wird das Halteelement 1 in die gewünschte Endposition bezüglich der zu haltenden Knochen gebracht, und es werden mit Hilfe der Bohrbuchsen 30 die Bohrungen in den Knochen erstellt. Falls nach dem Bohren eines Loches das entsprechende Befestigungselement 2 eingesetzt werden soll, bevor die anderen Löcher gebohrt werden, so kann die entsprechende Bohrbuchse 30 entfernt werden. Dabei werden die Verbindungsstege 32 zu anderen, noch eingesetzten Bohrbuchsen durchschnitten oder beispielsweise an einer Sollbruchstelle (nicht gezeichnet) abgebrochen.

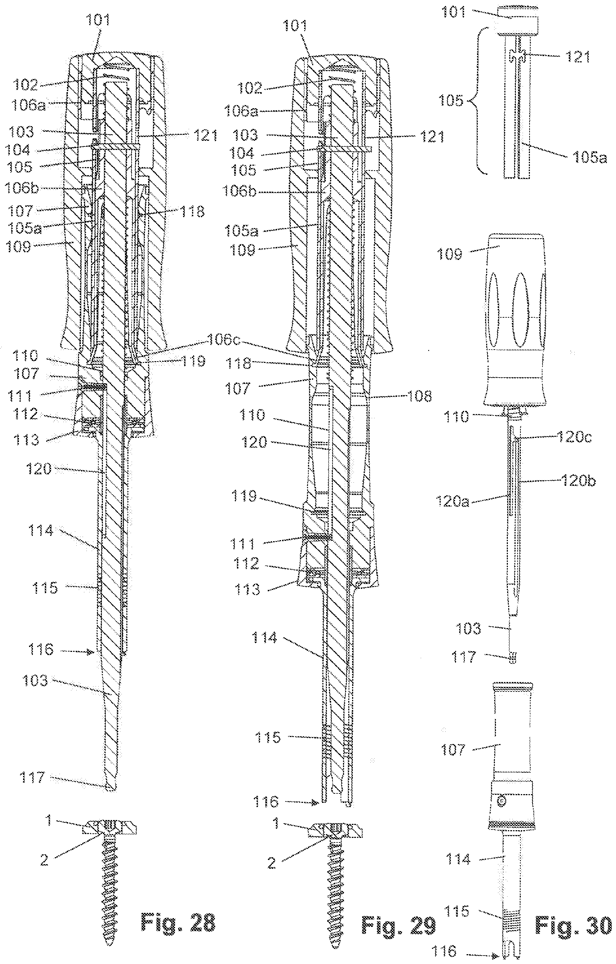

Zur Arbeit mit der Befestigungsvorrichtung, d.h. zum Drehen und insbesondere Schrauben des Befestigungselementes 2 und zum Drehen und damit Verriegeln oder Entriegeln des Ringes 12 kann ein Werkzeug verwendet werden, wie es in den **Figuren 28** bis **31** in verschiedenen Schnitten und Ansichten dargestellt ist. Das Werkzeug weist als Hauptbestandteile auf:
▪ einen inneren Schaft 103 mit einer inneren Werkzeugspitze 117. Diese ist beispielsweise korrespondierend zur Aufnahme 6 des Befestigungselementes 2 geformt. Die innere Werkzeugspitze 117 kann somit gemäss gängigen Typen von Schraubendrehern geformt sein, beispielsweise wie ein Schlitz-, Kreuz-, Phillips, Pozidriv. Sechskant oder Torx-Schraubendreher. Der innere Schaft 103 ist an einem Griff 109 montiert.
▪ einen äusseren Schaft 114 mit einer äusseren Werkzeugspitze 116, welche korrespondierend zu der oder den Öffnungen 17, 21 des Ringes 12 geformt sein kann, beispielsweise hinterschnittig, so dass sich die äussere Werkzeugspitze 116 in der oder den Öffnungen 17, 21 verhakt, wodurch ein Abrutschen des Werkzeuges verhindert wird und eine sichere Kraftübertragung respektive Drehomomentübertragung auf dem Ring möglich ist. Der äussere Schaft 114 ist an einer Teleskophülse 107 montiert.
Der äussere Schaft 114 und die Teleskophülse 107 sind miteinander bezüglich des inneren Schaftes 103 und des Griffes 109 entlang einer Längsachse verschiebbar, also in axialer Richtung. Der innere Schaft 103 verläuft dabei innerhalb des äusseren Schaftes 114, beispielsweise koaxial. In einer ersten Verschiebungslage (**Figur 28**) ist der äussere Schaft 114 eingezogen, so dass die innere Werkzeugspitze 117 verwendet werden kann. In einer zweiten Verschiebungslage (**Figur 29**) ist der äussere Schaft 114 ausgezogen, so dass die äussere Werkzeugspitze 116 verwendet werden kann.

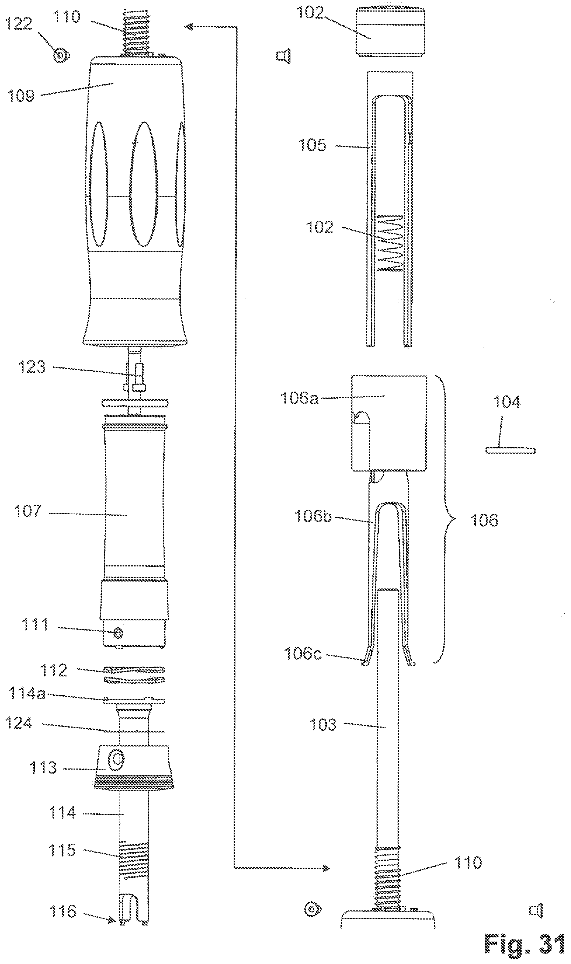

Zur Verriegelung in den beiden Verschiebungslagen ist ein Riegelelement 106 vorgesehen, zur Aufhebung der Verriegelung ein Auslöseelement 105, welches durch einen Auslöseknopf 101 betätigbar ist (**Figur 31**)**.** Das Riegelelement 106 weist einen Riegelkopf 106a sowie elastische Riegelzungen 106b mit Riegelrasten 106c an deren Ende. Die Riegelrasten 106c rasten von innen her an hinteren oder vorderen Rastnuten 118, 119 der Teleskophülse 107 ein. In der ersten Verschiebungslage (**Figur 28**) rasten sie in der vorderen Rastnut 119 ein, in der zweiten Verschiebungslage (**Figur 29**) in der hinteren Rastnut 118. Zum Lösen der Verriegelung wird der Auslöseknopf 101 gegen die Kraft einer Feder 102 nach innen gedrückt, wodurch sich das Auslöseelement 105 jeweils mit einem Auslösefinger 105a über die Riegelzungen 106b schiebt und die Riegelzungen 106b nach innen drückt, und damit auch die Riegelrasten 106c nach innen drückt und aus der Rastnut zieht.

Geschieht diese Freigabe in der ersten Verschiebungslage (**Figur 28**)**,** also bei eingezogenem äusseren Schaft 114, so wird die Teleskophülse 107 zusammen mit dem äusseren Schaft 114 durch eine Feder 110 nach vorne gestossen. Damit im Falle einer unbeabsichtigten Betätigung des Auslöseknopfes 101 während der Arbeit mit der inneren Werkzeugspitze 117 nicht die äussere Werkzeugspitze 116 nicht gegen beispielsweise die Platte oder den Ring schlägt, ist eine Bremse zur Begrenzung dieser Bewegung vorgesehen. Dazu kann die Innenseite der Teleskophülse 107, welche sich entlang den Riegelzungen 106b und Riegelrasten 106c bewegt, eine verengte Stelle oder Bremsstelle 108 aufweisen, welche entlang der Bewegungsrichtung der Riegelzungen 106b vor der hinteren Rastnut 118 befindet. Bewegt sich die Teleskophülse 107 nach vorne, so stossen die Riegelzungen 106b an der Bremsstelle 108 gegen die Innenseite der Teleskophülse 107, werden nach innen gestossen und bremsen dadurch die Bewegung der Teleskophülse 107 ab, bevor die die äussere Werkzeugspitze 116 in den Bereich der inneren Werkzeugspitze 117 gelangt. Um den äusseren Schaft 114 in die vorderste, ganz ausgezogene Verschiebungslage zu bringen, wird die Teleskophülse 1 07 von Hand nach vorne gezogen, bis die Riegelzungen 106b in der hinteren Rastnut 118 einrasten (**Figur 29**).

Bei der Arbeit mit dem Werkzeug ist ein Drehmoment vom Handgriff 109 an die jeweils aktive Werkzeugspitze 116, 117 zu übertragen.
▪ Ist die innere Werkzeugspitze 117 aktiv, so wird das Drehmoment über die folgenden Teile geleitet: Handgriff 109 - Schrauben oder Stifte 123 (in **Figur 28, 29** nicht sichtbar) - Riegelkopf 106a - Stift 104 - innerer Schaft 103 - innere Werkzeugspitze 117.
▪ Ist die äussere Werkzeugspitze 116 aktiv, so wird das Drehmoment vom inneren Schaft 103 ausgehend über die folgenden Teile weiter geleitet: innerer Schaft 103 - Schlitz 120 des inneren Schaftes - Stift 111 der Teleskophülse 107 - Teleskophülse 107 - Drehmomentbegrenzer 112 - äusserer Schaft 114 - äussere Werkzeugspitze 116.

Der genannte Schlitz 120 des inneren Schaftes verläuft in Längsrichtung des inneren Schaftes 103, so dass der Stift 111 der Teleskophülse 107 ungeachtet der Verschiebungslage in den Schlitz 120 hinein ragt.

Der genannte Drehmomentbegrenzer 112 ist zwischen einem vorderen Ende der Teleskophülse 107 und einem Endstück 114a des äusseren Schaftes 114 angeordnet. Das Endstück 114a wird mit einem Hülsenkopf 113, welcher an der Teleskophülse 107 befestigt ist, gegen den Drehmomentbegrenzer 112 gezogen. Dabei kann ein Gleitelement 124 zur Verringerung der Reibung zwischen Hülsenkopf 113 und Endstück 114a angeordnet sein.

Das Werkzeug kann werkzeugfrei zerlegbar sein, um hygienischen Vorschriften für medizinisch verwendete Geräte zu genügen (**Figur 30**). Damit können einerseits das Auslöseelement 105 mit dem Auslöseknopf 101 und andererseits die Teleskophülse 107 mit dem äusseren Schaft 114 abgetrennt werden.
▪ Um die Teleskophülse 107 abzutrennen weist der Schlitz 120 des inneren Schaftes zwei Abschnitte auf, in welchen der Stift 111 gleiten kann: einen ersten Abschnitt 120a zur Drehmomentübertragung, mit einer Länge entsprechend den beiden Verschiebungslagen, und einen zweiten Abschnitt 120b zur Demontage der Teleskophülse 107. Die beiden Abschnitte 120a, 120b laufen parallel zueinander, in Umfangsrichtung des inneren Schaftes 103 gegeneinander versetzt. Der erste Abschnitt 120a ist in seiner Länge begrenzt und begrenzt dadurch die Bewegung der Teleskophülse 107 entlang des inneren Schaftes 103. Der erste Abschnitt 120b hingegen führt weiter zur Spitze des inneren Schaftes 103 und ist gegen die Spitze hin offen. An einer Übergangsstelle 120c läuft der zweite Abschnitt 120b in den ersten hinein. Somit kann, zur Demontage die Teleskophülse 107 durch Verschiebung in axialer Richtung in eine Verschiebungslage gebracht werden, in welcher der Stift 111 bei der Übergangsstelle 120c liegt. Dann kann die Teleskophülse 107 um die axiale Richtung verdreht werden, wodurch der Stift 111 in den zweiten Abschnitt gelangt und die Teleskophülse 107 vom inneren Schaft 103 abgezogen werden kann. Gemäss einer Ausführungsform ist zudem an der Übergangsstelle 120c der Schlitz 120 weniger tief als im ersten Abschnitt 120a und ist der Stift 111 durch eine Feder in den Schlitz 120 hinein gedrückt. Dann muss beim Verdrehen der Teleskophülse 107 eine zusätzliche Kraft aufgewendet werden, um den Stift 111 nach aussen zu stossen. Dies ergibt eine Sicherheit gegen unbeabsichtigtes Demontieren.
▪ Um das Auslöseelement 105 abzutrennen liegt in den Auslösefingern ein Schlitz 121 vor, in welchen beispielsweise der Stift 104 in radialer Richtung ragt. Beim Drücken des Auslöseelementes 105 bewegt sich der Schlitz 121 entlang des Stiftes 104, wobei die Bewegung des Auslöseelementes 105 in Längsrichtung des Werkzeuges durch die Länge des Schlitzes 121 begrenzt ist. Der Schlitz 121 weist eine seitliche Öffnung so dass das Auslöseelement 105, wenn sich diese Öffnung auf der Höhe des Stiftes 104 befindet, um die Längsachse des Werkzeuges drehbar ist, wonach das Auslöseelemente 105 herausgezogen werden kann.

In der bisherigen Beschreibung war jeweils nur von einem Schlitz 120 des inneren Schaftes und einem darin laufenden Stift 111 die Rede. Es versteht sich, dass zur besseren Kraftübertragung auch zwei, drei oder mehr Schlitze 120 und korrespondierende Stifte 111 um den Umfang des Werkzeuges verteilt angeordnet sein können.

Da gemäss einer Ausführungsform die äussere Werkzeugspitze 116 sich mit beispielsweise einem Ring 12 eines Befestigungselementes 2 verhaken kann, können aufgrund des Hebelarmes, den das Werkzeug bildet, grosse Kräfte am Befestigungselement 2 auftreten. Dies kann zu Schäden und/oder zur Verschiebung des Befestigungselementes oder damit verbundenen gebrochenen Knochen führen. Um dies zu verhindern, kann der äussere Schaft 114 biegeelastisch und drehsteif ausgebildet sein oder einen elastischen Bereich 115 aufweisen, welcher gegenüber den anderen Bereichen des äusseren Schaftes 114 eine höhere Elastizität, insbesondere Biegeelastizität aufweist, jedoch drehsteif ist, um ein Drehmoment übertragen zu können. Beispielsweise können dazu nach Art einer Wendelkupplung wendelförmige Einschnitte am äusseren Schaft 114 vorliegen, oder es kann eine Drehfeder eingebaut werden. Damit der innere Schaft 103 die Biegung des äusseren Schaftes 114 nicht blockiert, ist der innere Schaft 103 auf der Höhe des elastischen Bereiches 115 (bei ausgezogenem äusserem Schaft) und weiter vorne, zur inneren Werkzeugspitze 117 hin, verjüngt ausgebildet

Der Drehmomentbegrenzer 112 kann zwei gegeneinander gepresste federnde Elemente aufweisen, welche eine das Drehmoment durch Reibung erzeugen. Die **Figuren 32** bis **33** zeigen alternativ die Funktionsweise einer Variante für einen asymmetrisch wirkenden Drehmomentbegrenzer 112. Gezeigt ist jeweils ein Abschnitt eines oberen Ringes 112a und eines unteren Ringes 112b, welche durch eine Kraft F in axialer Richtung, also parallel zur Längsrichtung des Werkzeuges gegeneinander gedrückt werden. Die beiden Ringe 112a, 112b weisen symmetrisch zueinander geformte Vorsprünge 112c auf. Mehrere solcher Vorsprünge 112c sind in Umfangsrichtung regelmässig um die Ringe 112a, 112b verteilt. Zwischen den beiden Ringen sind Kugeln oder Rollen 112d angeordnet. Verdrehen sich die Ringe 112a, 112b um die axiale Richtung gegeneinander, so rollen sie über die Rollen 112d aneinander ab, bis die Rollen 112d an den Vorsprüngen 112c anstehen. Abhängig von einer Steilheit von Flanken der Vorsprünge ist an jedem Vorsprung in horizontaler Richtung ein Mehrfaches oder ein Bruchteil der Kraft F aufzuwenden, um die Ringe 112a, 112b voneinander zu entfernen. Daraus ergibt sich ein entsprechendes maximales Drehmoment, bei welchem der Drehmomentbegrenzer durchdreht. Durch unterschiedliche Steilheit der Flanken an den beiden Seiten der Vorsprünge 112c ist das maximale Drehmoment in die beiden Drehrichtungen separat wählbar. Beim gezeigten Beispiel beginnt in **Figur 32** bei einem ersten Drehmoment das Anheben des oberen Ringes 112a und damit das Durchdrehen. Entsprechend dem Profilverlauf der Vorsprünge 112c nimmt in der Lage gemäss **Figur 33** das durch den Begrenzer erzeugte Gegenmoment bereits wieder ab. Bei einer Drehbewegung in Gegenrichtung ist entsprechend der steileren Flanke auf der Gegenseite (**Figur 34**) das zum Durchdrehen in Gegenrichtung erforderliche maximale Moment wesentlich höher.

Für das beschriebene Werkzeug ist das Maximalmoment beim Verriegeln der Befestigungsvorrichtung (beispielsweise durch Drehung im Uhrzeigersinn) durch die Konstruktion und die Materialien der Befestigungsvorrichtung bestimmt. Zum Entriegeln wird hingegen ein höheres Maximalmoment eingestellt.

## Patentansprüche

1. Eine **Befestigungsvorrichtung für chirurgische Haltesysteme,** aufweisend ein Halteelement (1) und ein Befestigungselement (2) welche mechanisch miteinander verbindbar sind,
wobei das Halteelement (1) eine Gelenkpfanne (8) und das Befestigungselement (2) einen Gelenkkopf (3) aufweist, wobei die Gelenkpfanne (8) und der Gelenkkopf (3) zueinander korrespondierend geformt sind und ein Kugelgelenk bilden, und die Bewegung des Kugelgelenkes mittels eines Klemmelementes arretierbar ist,
wobei das Klemmelement ein Exzenter-Ring (12) ist, welcher beim Verdrehen den Gelenkkopf (3) bezüglich der Gelenkpfanne (8) verklemmt,
wobei die Gelenkpfanne (8) eine Gelenkinnenfläche (15) des Kugelgelenkes und der Gelenkkopf (3) eine Gelenkaussenfläche (9) des Kugelgelenkes definieren; **dadurch gekennzeichnet, dass**
wenn der Exzenter-Ring (12) den Gelenkkopf (3) bezüglich der Gelenkpfanne (8) verklemmt, durch die Form von mindestens einem der drei Elemente Gelenkpfanne (8), Gelenkkopf (3) und Exzenter-Ring (12) die Lage von drei Kontaktstellen des Kugelgelenkes, welche das Kugelgelenk arretieren, eindeutig definiert ist.

2. Befestigungsvorrichtung gemäss Anspruch 1, wobei der Exzenter-Ring (12) am Halteelement (1) drehbar angeordnet ist und bei Verdrehung des Exzenter-Ringes (12) bezüglich des Halteelementes (1) der Exzenter-Ring (12) selber oder ein vom Exzenter-Ring (12) bewegtes Zwischenelement gegen die Gelenkaussenfläche (9) gedrückt wird.

3. Befestigungsvorrichtung gemäss Anspruch 1, wobei der Exzenter-Ring (12) am Befestigungselement (2) drehbar angeordnet ist und bei Verdrehung des Exzenter-Ringes (12) bezüglich des Befestigungselementes (2) der Exzenter-Ring (12) selber oder ein vom Exzenter-Ring (12) bewegtes Zwischenelement (41) gegen die Gelenkinnenfläche (15) gedrückt wird.

4. Befestigungsvorrichtung gemäss einem der Ansprüche 1 bis 3, wobei
▪ entweder die Gelenkinnenfläche (15) genau zwei oder genau drei nach innen vorstehende Kontaktbereiche (16) aufweist,
▪ oder die Gelenkaussenfläche (9) genau zwei oder genau drei nach aussen vorstehende Kontaktbereiche aufweist,
und dabei, wenn der Exzenter-Ring (12) den Gelenkkopf (3) bezüglich der Gelenkpfanne (8) verklemmt, aber keine Verformung an diesen Teilen stattgefunden hat, genau drei oder vier Kontaktstellen zwischen der Gelenkinnenfläche (15) und der Gelenkaussenfläche (9) vorliegen.

5. Befestigungsvorrichtung gemäss einem der Ansprüche 1 bis 4, wobei die Gelenkinnenfläche (15) genau zwei oder genau drei nach innen vorstehende Kontaktbereiche (16) aufweist und insbesondere die Gelenkaussenfläche (9) keine nach aussen vorstehende Kontaktbereiche aufweist.

6. Befestigungsvorrichtung gemäss einem der Ansprüche 1 bis 5, wobei der Exzenter-Ring (12) zwei vorstehende Kontaktbereiche (16) aufweist.

7. Befestigungsvorrichtung gemäss einem der Ansprüche 1 bis 5, wobei zwei der definierten Kontaktstellen an zwei Kontaktbereichen (16) durch Verformen des Exzenter-Ringes (12) entstehen, insbesondere an zwei Seiten einer entlang des Umfanges des Exzenter-Ringes (12) verlaufenden biegeschwachen Stelle.

8. Befestigungsvorrichtung gemäss Anspruch 6 oder 7, wobei Verbindungslinien der beiden Kontaktbereiche (16) zur Mittelachse des Exzenter-Ringes (12) einen Winkel von weniger als 170° bilden, insbesondere einen Winkel von weniger als 100°.

9. Befestigungsvorrichtung gemäss einem der Ansprüche 1 bis 8, wobei die Gelenkinnenfläche (15) an einer dem Exzenter-Ring (12) gegenüberliegenden Seite des Halteelementes 1 drei vorstehende Kontaktbereiche (16) aufweist, welche im wesentlichen gleichmässig am Umfang einer Durchführung (20) für das Befestigungselement (2) angeordnet sind.

10. Befestigungsvorrichtung gemäss einem der Ansprüche 1 bis 4, wobei die Gelenkaussenfläche (9) genau zwei oder genau drei nach aussen vorstehende Kontaktbereiche aufweist und insbesondere die Gelenkinnenfläche (15) keine nach innen vorstehende Kontaktbereiche (16) aufweist.

11. Befestigungsvorrichtung gemäss einem der Ansprüche 1, 3, 4, 9 oder 10, wobei der Exzenter-Ring (12) am Befestigungselement (2) drehbar angeordnet ist und der Gelenkkopf (3) ein Zwischenelement (41) aufweist, welches durch Verdrehen des Exzenter-Ringes (12) gegen die Gelenkpfanne (8) des Halteelementes (1) drückbar ist und dadurch eine erste Kontaktstelle bildet und das Kugelgelenk arretiert, und der Gelenkkopf (3) mindestens zwei Segmente (42) aufweist, welche dem Zwischenelement (41) bezüglich des Gelenkkopfes (3) gegenüberliegend angeordnet sind, wobei jedes der mindestens zwei Segmente (42) einen Kontaktbereich (16) und damit eine weitere Kontaktstelle des Kugelgelenkes definiert.

12. Befestigungsvorrichtung gemäss einem der Ansprüche 1, 3, 4, 9 oder 10, wobei der Exzenter-Ring (12) am Befestigungselement (2) drehbar angeordnet ist und ein Abschnitt des Exzenter-Ringes (12) durch Verdrehen des Exzenter-Ringes (12) gegen die Gelenkpfanne (8) des Halteelementes (1) drückbar ist und dadurch eine erste Kontaktstelle bildet und das Kugelgelenk arretiert, und der Gelenkkopf (3) mindestens zwei Segmente (42) aufweist, welche der ersten Kontaktstelle bezüglich des Gelenkkopfes (3) gegenüberliegend angeordnet sind, wobei jedes der mindestens zwei Segmente (42) einen Kontaktbereich (16) und damit eine weitere Kontaktstelle des Kugelgelenkes definiert.

13. Befestigungsvorrichtung gemäss einem der vorangehenden Ansprüche, wobei der kleinste Abstand zwischen den drei definierten Kontaktstellen mindestens das 0.8-fache des Durchmessers des Gelenkkopfes (3) beträgt.

14. Befestigungsvorrichtung gemäss einem der vorangehenden Ansprüche, aufweisend ein Einrastelement (19) zur einrastenden Festhalten des Exzenter-Ringes (12) bezüglich Verdrehung gegenüber dem Halteelement (1).

15. Befestigungsvorrichtung gemäss einem der vorangehenden Ansprüche, wobei der Exzenter-Ring (12) mindestens eine hinterschnittige Öffnung (17) zum Einführen eines Werkzeuges zum Verdrehen des Exzenter-Ringes (12) aufweist.

## Claims

1. A **fixing device for surgical holding systems,** comprising a holding element (1) and a fixing element (2) which are mechanically connectable to one another, wherein the holding element (1) comprises a joint socket (8) and the fixing element (2) comprises an articulated head (3) wherein the joint socket (8) and the articulated head (3) are shaped corresponding to one another and form a ball and socket joint, and the movement of the ball and socket joint is lockable by means of a clamping element,
wherein the clamping element is an eccentric ring (12), which when turned clamps the articulated head (3) relative to the joint socket (8),
wherein the joint socket (8) defines a joint inside surface (15) of the ball and socket joint and the articulated head (3) defines a joint outside surface (9) of the ball and socket joint; **characterised in that**
when the eccentric ring (12) clamps the articulated head (3) relative to the joint socket (8), by the shape of at least one of the three elements joint socket (8), articulated head (3) and eccentric ring (12) the position of three contact points of the ball and socket joint, which lock the ball and socket joint, is unequivocally defined.

2. A fixing device in accordance with claim 1, wherein the eccentric ring (12) is arranged as rotatable on the holding element (1) and when the eccentric ring (12) is rotated relative to the holding element (1) the eccentric ring (12) itself or an intermediate element moved by the eccentric ring (12) is pressed against the joint outside surface (9).

3. A fixing device in accordance with claim 1, wherein the eccentric ring (12) is arranged as rotatable on the fixing element (2) and when the eccentric ring (12) is rotated relative to the fixing element (2) the eccentric ring (12) itself or an intermediate element (41) moved by the eccentric ring (12) is pressed against the joint inside surface (15).

4. A fixing device in accordance with one of the claims 1 to 3, wherein
▪ either the joint inside surface (15) comprises exactly two or exactly three contact areas (16) protruding towards the inside,
▪ or the joint outside surface (9) comprises exactly two or exactly three contact areas protruding towards the outside,
and in that, when the eccentric ring (12) clamps the articulated head (3) relative to the joint socket (8), but no deformation has taken place on these components, exactly three or four contact points between the joint inside surface (15) and the joint outside surface (9) are present.

5. A fixing device in accordance with one of the claims 1 to 4, wherein the joint inside surface (15) comprises exactly two or exactly three contact areas (16) protruding towards the inside and in particular the joint outside surface (9) does not comprise any contact areas protruding towards the outside.

6. A fixing device in accordance with one of the claims 1 to 5, wherein the eccentric ring (12) comprises two protruding contact areas (16).

7. A fixing device in accordance with one of the claims 1 to 5, wherein two of the defined contact points on two contact areas (16) are produced by deforming the eccentric ring (12), in particular at two sides of a flexurally weak region extending along the circumference of the eccentric ring (12).

8. A fixing device in accordance with claim 6 or 7, wherein connecting lines from the two contact areas (16) to the centre axis of the eccentric ring (12) form an angle of less than 170°, in particular an angle of less than 100°.

9. A fixing device in accordance with one of the claims 1 to 8, wherein the joint inside surface (15) on a side of the holding element (1) opposite the eccentric ring (12) comprises three protruding contact areas (16), which essentially are arranged uniformly on the circumference of a passage (20) for the fixing element (2).

10. A fixing device in accordance with one of the claims 1 to 4, wherein the joint outside surface (9) comprises exactly two or exactly three contact areas protruding towards the outside and in particular the joint inside surface (15) does not comprise any contact areas (16) protruding towards the inside.

11. A fixing device in accordance with one of the claims 1, 3, 4, 9 or 10, wherein the eccentric ring (12) is arranged as rotatable on the fixing element (2) and the articulated head (3) comprises an intermediate element (41), which by rotating the eccentric ring (12) is pressable against the joint socket (8) of the holding element (1) and as a result forms a first contact point and locks the ball and socket joint, and the articulated head (3) comprises at least two segments (42) which are arranged relative to the articulated head (3) opposite the intermediate element (41), wherein each one of the at least two segments (42) defines a contact area (16) and with this a further contact point of the ball and socket joint.

12. A fixing device in accordance with one of the claims 1, 3, 4, 9 or 10, wherein the eccentric ring (12) is arranged as rotatable on the fixing element (2) and a section of the eccentric ring (12) by rotating the eccentric ring (12) is pressable against the joint socket (8) of the holding element (1) and as a result forms a first contact point and locks the ball and socket joint, and the articulated head (3) comprises at least two segments (42), which are arranged opposite the first contact point relative to the articulated head (3), wherein each one of the at least two segments (42) defines a contact area (16) and with this a further contact point of the ball and socket joint.

13. A fixing device in accordance with one of the preceding claims, wherein the smallest distance between the three defined contact points amounts to at least 0.8 times the diameter of the articulated head (3).

14. A fixing device in accordance with one of the preceding claims, comprising a latching element (19) for holding the eccentric ring (12) by latching (engaging, catching) with respect to rotating relative to the holding element (1).

15. A fixing device in accordance with one of the preceding claims, wherein the eccentric ring (12) comprises at least one undercut opening (17) for introducing a tool for rotating the eccentric ring (12).

## Revendications

1. Ensemble de fixation pour systèmes chirurgicaux de maintien, présentant un élément de maintien (1) et un élément de fixation (2) qui peuvent être reliés l'un à l'autre mécaniquement,
l'élément de maintien (1) présentant une cuvette (8) d'articulation et l'élément de fixation (2) une tête (3) d'articulation, la cuvette (8) d'articulation et la tête (3) d'articulation étant configurées en correspondance mutuelle et formant une articulation sphérique, le déplacement de l'articulation sphérique pouvant être bloqué par un élément de serrage,
l'élément de serrage étant une bague excentrique (12) qui se serre lors de la rotation de la tête (3) de l'articulation par rapport à la cuvette (8) de l'articulation,
la cuvette (8) de l'articulation définissant une surface intérieure (15) de l'articulation sphérique et la tête (3) de l'articulation une surface extérieure (9) de l'articulation sphérique,
**caractérisé en ce que**
lorsque la bague excentrique (12) serre la tête (3) de l'articulation par rapport à la cuvette (8) de l'articulation, la position de trois emplacements de contact de l'articulation sphérique qui bloquent l'articulation sphérique est définie de manière univoque par la forme d'au moins l'un des trois éléments que sont la cuvette (8) de l'articulation, la tête (3) de l'articulation et la bague excentrique (12) .

2. Ensemble de fixation selon la revendication 1, dans lequel la bague excentrique (12) est disposée à rotation sur l'élément de maintien (1) et, lors de la rotation de la bague excentrique (12) par rapport à l'élément de maintien (1), la bague excentrique (12) elle-même ou un élément intermédiaire déplacé par la bague excentrique (12) sont repoussés contre la surface extérieure (9).

3. Ensemble de fixation selon la revendication 1, dans lequel la bague excentrique (12) est disposée à rotation sur le l'élément de fixation (2) et, lors de la rotation de la bague excentrique (12) par rapport au l'élément de fixation (2), la bague excentrique (12) elle-même ou un élément intermédiaire déplacé par la bague excentrique (12) sont repoussés contre la surface intérieure (15) .

4. Ensemble de fixation selon l'une des revendications 1 à 3, dans lequel
soit la surface intérieure (15) de l'articulation présente exactement deux ou exactement trois parties de contact (16) débordant vers l'intérieur,
soit la surface extérieure (9) de l'articulation présente exactement deux ou exactement trois parties de contact débordant vers l'extérieur et ainsi, lorsque la bague excentrique (12) serre la tête (3) de l'articulation par rapport à la cuvette (8) de l'articulation mais qu'aucune déformation de ces pièces n'a eu lieu, exactement trois ou quatre emplacements de contact sont formés entre la surface intérieure (15) de l'articulation et la surface extérieure (9) de l'articulation.

5. Ensemble de fixation selon l'une des revendications 1 à 4, dans lequel la surface intérieure (15) de l'articulation présente exactement deux ou exactement trois parties de contact (16) débordant vers l'intérieur, et en particulier la surface extérieure (9) de l'articulation ne présente pas de partie de contact débordant vers l'extérieur.

6. Ensemble de fixation selon l'une des revendications 1 à 5, dans lequel la bague excentrique (12) présente deux parties de contact (16) en saillie.

7. Ensemble de fixation selon l'une des revendications 1 à 5, dans lequel deux des emplacements de contact définis sont formés sur deux parties de contact (16) par déformation de la bague excentrique (12), en particulier sur deux côtés d'un emplacement fléchissant qui s'étend le long de la périphérie de la bague excentrique (12).

8. Ensemble de fixation selon les revendications 6 ou 7, dans lequel des lignes de liaison entre les deux parties de contact (16) et l'axe central de la bague excentrique (12) forment un angle inférieur à 170° et en particulier un angle inférieur à 100°.

9. Ensemble de fixation selon l'une des revendications 1 à 8, dans lequel la surface intérieure (15) de l'articulation présente sur un côté de l'élément de maintien (1) situé face à la bague excentrique (12) trois parties de contact (16) en saillie qui sont disposées de manière essentiellement régulière à la périphérie d'un passage (20) prévu pour l'élément de fixation (2).

10. Ensemble de fixation selon l'une des revendications 1 à 4, dans lequel la surface extérieure (9) de l'articulation présente exactement deux ou exactement trois parties de contact débordant vers l'extérieur et en particulier la surface intérieure (15) de l'articulation ne présente pas de partie de contact (16) en saillie vers l'intérieur.

11. Ensemble de fixation selon l'une des revendications 1, 3, 4, 9 ou 10, dans lequel la bague excentrique (12) est disposée à rotation sur le l'élément de fixation (2) et la tête (3) de l'articulation présente un élément intermédiaire (41) qui peut être repoussé contre la cuvette (8) de l'articulation de l'élément de maintien (1) par rotation de la bague excentrique (12) pour ainsi former un premier emplacement de contact et bloquer l'articulation sphérique, la tête (3) de l'articulation présentant au moins deux segments (42) disposés face à l'élément intermédiaire (41) par rapport à la tête (3) de l'articulation, chacun des deux ou plusieurs segments (42) définissant une partie de contact (16) et donc un autre emplacement de contact de l'articulation sphérique.

12. Ensemble de fixation selon l'une des revendications 1, 3, 4, 9 ou 10, dans lequel la bague excentrique (12) est disposée à rotation sur l'élément de fixation (2) et une partie de la bague excentrique (12) peut être repoussée contre la cuvette (8) de l'articulation de l'élément de maintien (1) par rotation de la bague excentrique (12) pour ainsi former un premier emplacement de contact et bloquer l'articulation sphérique, la tête (3) de l'articulation présentant au moins deux segments (42) disposés face au premier emplacement de contact par rapport à la tête (3) de l'articulation, chacun des deux ou plusieurs segments (42) définissant une partie de contact (16) et donc un autre emplacement de contact de l'articulation sphérique.

13. Ensemble de fixation selon l'une des revendications précédentes, dans lequel la plus petite distance entre les trois emplacements de contact définis représente au moins 0,8 fois le diamètre de la tête (3) de l'articulation.

14. Ensemble de fixation selon l'une des revendications précédentes, présentant un élément d'encliquetage (19) qui empêche par encliquetage la bague excentrique (12) de tourner par rapport à l'élément de maintien (1).

15. Ensemble de fixation selon l'une des revendications précédentes, dans lequel la bague excentrique (12) présente au moins une ouverture (17) en contre-dépouille qui permet d'insérer un outil permettant de faire tourner la bague excentrique (12).
